# EUROPEAN PATENT APPLICATION

(11) **EP 3 056 205 A1**
(43) Date of publication of application: **17.08.2016**
(21) Application number: 16159268.8
(22) Date of filing: 08.07.2011
(51) Int. Cl.: A61K 31/497, A61K 31/47

(54) **5-CHLORO-4-HYDROXY-1-METHYL-2-OXO-N-PHENYL-1,2-DIHYDROQUINOLINE-3-CARBOXAMIDE, SALTS AND USES THEREOF**

(30) Priority: 09.07.2010 US 399264 P
(62) Divisional of application: 11804416.3
(71) Applicant: TEVA PHARMACEUTICAL INDUSTRIES, LTD., 49131 Petah Tiqva (IL)
(72) Inventor: FRISTEDT, Ulf, Tomas, S-256 56 Helsingborg (SE)
(74) Representative: D Young & Co LLP

(57) **Abstract**

The subject invention provides 5-chloro-4-hydroxy-1-methyl-2-oxo-N-phenyl-1,2-dihydroquinoline-3-carboxamide, its salts and uses.

## Description

This application claims priority of U.S. Provisional Application No. 61/399,264, filed July 9, 2010, the contents of which are hereby incorporated by reference.

Throughout this application various publications, published patent applications, and patents are referenced. The disclosures of these documents in their entireties are hereby incorporated by reference into this application in order to more fully describe the state of the art to which this invention pertains.

### Background of the Invention

Laquinimod is a compound which has been shown to be effective in the acute experimental autoimmune encephalomyelitis (aEAE) model (U.S. Patent No. 6,077,851). Its chemical name is N-ethyl-N-phenyl-1,2-dihydro-4-hydroxy-5-chloro-l-methyl-2-oxoquinoline-3-carhoxamide, and its Chemical Registry number is 248281-84-7. The processes of synthesis of laquinimod and the preparation of its sodium salt are disclosed in U.S. Patent No. 6,077,851. An additional process of synthesis of laquinimod is disclosed in U.S. Patent No. 6,875,869.

Pharmaceutical compositions comprising laquinimod sodium are disclosed in PCT International Application Publication No. WO 2005/074899.

Laquinimod sodium is a novel synthetic compound with high oral bioavailability, which has been suggested as an oral formulation for the treatment of Multiple Sclerosis (MS). (Polman, C. et al., (2005) 'Treatment with laquinimod reduces development of active MRI lesions in relapsing MS", Neurology. 64:987-991; Sandberg-Wollheim M, et al. (2005) "48-week open safety study with high-dose oral laquinimod in patients", Mult Scler. 11:5154) Studies have also shown that laquinimod can reduce development of active MRI lesions in relapsing MS. (Polman, C. ct at., (2005) "Treatment with laquinimod reduces development of active MRI lesions in relapsing MS", Neurology. 64:987-991).

In order to prepare laquinimod as a pharmaceutical drug product, processes are required which take into consideration of the impurities disclosed herein.

### Summary of the Invention

An undesirable impurity has been identified in laquinimod preparations.

The subject invention provides a composition comprising a compound having the structure: 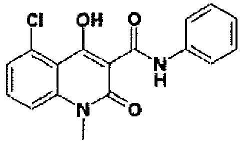 in an amount from more than 3ppm to less than 90 wt%, based on the total weight of the composition, and a carrier.

The subject invention also provides a pharmaceutical composition comprising a mixture of:
a) laquinimod or a pharmaceutically acceptable salt thereof;
b) at least one pharmaceutically acceptable carrier; and
c) a compound having the structure: 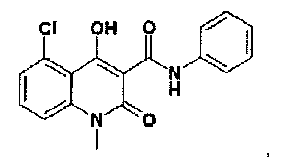 present in an amount less than 0.1% based on the combined weight of the compound and laquinimod.

The subject invention further provides a process for preparing the pharmaceutical composition described herein, the process comprises:
a) obtaining a batch of laquinimod or a pharmaceutically acceptable salt thereof;
b) determining by apparatus the total amount of 5-chloro-4-hydroxy-1-methyl-2-oxo-N-phenyl-1,2-dittydroquinoline-3-carboxamidc present in the batch of laquinimod or a pharmaceutically acceptable salt thereof; and
c) preparing the pharmaceutical composition using the batch only if the batch is determined to have less than 0.10% by weight of 5-chloro-A-hydroxy-1-mathyl-2-oxo-N-phenyM,2-dihydroquinoline-3-carboxamide.

The subject invention yet further provides a process for producing a validated batch of a pharmaceutical composition containing laquinimod or a pharmaceutically acceptable salt thereof and at least one pharmaceutically acceptable carrier for distribution, the process comprises:
a) obtaining a batch of the pharmaceutical composition;
b) determining by apparatus the total amount of 5-chloro-4-hydroxy-1-metbyl-2-oxo-N-phenyl-1,2-dihydroquinoline-3-carboxamide in a sample of the batch; and
c) validating the batch for distribution only if the sample of the batch is determined to contain less than 0.1% by weight of 5-chloro-4-hydroxy-1-methyl-2-oxo-N-phenyl-1,2-dihydroquinoline-3-carboxamide relative to the combined weight of laquinimod and 5-chloro-4-hydrozy-1-methyl-2-oxo-N-phenyl-1,2-dihydroquinoline-3-carboxamide.

The subject invention yet further provides a process for producing laquinimod or a pharmaceutically acceptable salt thereof, the process comprises:
a) obtaining a batch of N-ethylaniline;
b) determining by apparatus the total amount of aniline in the batch of N-ethylaniline; and
c) preparing laquinimod or a pharmaceutically acceptable salt thereof using the batch of N-ethylaniline only if the batch of N-ethylaniline is determined to have less than 0.5% aniline by weight.

The subject invention yet further provides a process for producing laquinimod or a pharmaceutically acceptable salt thereof, the process comprises:
a) obtaining a batch of N-ethylaniline;
b) purifying the batch of N-ethylaniline by separating aniline from the batch of N-ethylaniline; and
c) preparing laquinimod or a pharmaceutically acceptable salt thereof using the purified batch of N-ethylaniline from step b).

The subject invention yet further provides a process for producing laquinimod or a pharmaceutically acceptable salt thereof, the process comprises:
a) obtaining a batch of N-ethylaniline containing less than 0.5% aniline by weight: and
b) preparing laquinimod or a pharmaceutically acceptable salt thereof using the batch of N-ethylaniline.

The subject invention yet further provides a process for preparing 5-chloro-4-ltydroxy-1-methyl-2-oxo-N-phenyl-1,2-dihydroquinoline-3-carboxamide, the process comprises:
a) reacting 5-chloro-1,2-dihydro-4-hydroxy-f-inethyl-2-oxo-quinoline-3-carboxylic acid methyl ester and aniline under suitable conditions; and
b) obtaining 5-chloro-4-hydroxy-1-methyl-2-oxo-N-phenyl-1,2-dihydroquinoline-3-carboxamide from the reaction.

### Brief Description of the Figures

Figure 1 is the HPLC chromatogram of a sample of laquinimod containing DELAQ impurity using HPLC analysis Condition 1.
Figure 2 is the HPLC chromatogram of a sample of laquinimod containing DELAQ impurity using HPLC analysis Condition 2.
Figure 3 is the HPLC chromatogram of a sample of N-ethylaniline (NEA) which contains aniline impurity.

### Detailed Description of the Invention

The subject invention provides a composition comprising a compound having the structure: 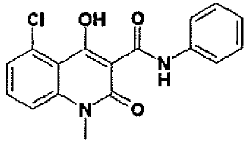 in an amount from more than 3ppm to less than 90 wt%, based on the total weight of the composition, and a carrier.

The subject invention also provides a pharmaceutical composition comprising a mixture of:
a) laquinimod or a pharmaceutically acceptable salt thereof;
b) at least one pharmaceutically acceptable carrier; and
c) a compound having the structure: 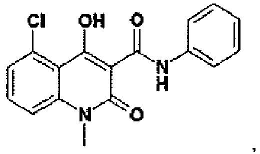 present in an amount less than 0.1% based on the combined weight of the compound and laquinimod.

In an embodiment of the pharmaceutical composition, the compound is present in an amount less than 3 ppm or less than 2 ppm based on the combined weight of the compound and laquinimod.

In another embodiment of the pharmaceutical composition, the pharmaceutical composition is in the form of a tablet.

The subject invention further provides a process for preparing the pharmaceutical composition described herein, the process comprises:
a) obtaining a batch of laquinimod or a pharmaceutically acceptable salt thereof;
b) determining by apparatus the total amount of 5-chloro-4-hydroxy-1-methyl-2-oxoN-phenyl-1,2-dihydroquinoline-3-carboxamide present in the batch of laquinimod or a pharmaceutically acceptable salt thereof; and
c) preparing the pharmaceutical composition using the batch only if the batch is determined to have less than 0.10% by weight of 5-chloro-4-hydroxy-1-methyl-2-oxo-N-phenyl-1,2-dihydroquinoline-3-carboxamide.

The subject invention yet further provides a process for producing a validated batch of a pharmaceutical composition containing laquinimod or a pharmaceutically acceptable salt thereof and at least one pharmaceutically acceptable carrier for distribution, the process comprises:
a) obtaining a batch of the pharmaceutical composition;
b) determining by apparatus the total amount of 5-chloro-4-hydroxy-1-methyl-2-oxoN-phenyl-1,2-dihydroquinoline-3-carboxamide in a sample of the batch; and
c) validating the batch for distribution only if the sample of the batch is determined to contain less than 0.1% by weight of 5-chloro-4-hydroxy-1-mothyl-2-oxo-N-phenyl-1,2-dihydroquinoline-3-carboxamide relative to the combined weight of laquinimod and 5-chloro-4-hydroxy-1-methyl-2-oxo-N-phenyl-1,2-dihydroquinoline-3-carboxamide.

The subject invention yet further provides a process for producing laquinimod or a pharmaceutically acceptable salt thereof, the process comprises:
a) obtaining a batch of N-ethylaniline;
b) determining by apparatus the total amount of aniline in the batch of N-ethylaniline; and
c) preparing laquinimod or a pharmaceutically acceptable salt thereof using the batch of N-ethylaniline only if the batch of N-ethylaniline is determined to have less than 0.5% aniline by weight.

The subject invention yet further provides a process for producing laquinimod or a pharmaceutically acceptable salt thereof, the process comprises:
a) obtaining a batch of N-ethylaniline;
b) purifying the batch of N-ethylaniline by separating aniline from the batch of N-ethylaniline; and
c) preparing laquinimod or a pharmaceutically acceptable salt thereof using the purified batch of N-ethylaniline from step b).

The subject invention yet further provides a process for producing laquinimod or a pharmaceutically acceptable salt thereof, the process comprises:
a) obtaining a batch of N-ethylaniline containing less than 0.5% aniline by weight; and
b) preparing laquinimod or a pharmaceutically acceptable salt thereof using the batch of N-ethylaniline.

The subject invention yet further provides a process for preparing 5-chloro-4-hydroxy-1-methyl-2-oxo-N-phenyl-1,2-dihydroquinoline-3-carboxamide, the process comprises:
a) reacting 5-chloro-1,2-dihydro-4-hydroxy-1-methyl-2-oxo-quinoline-3-carboxylic acid methyl ester and aniline under suitable conditions; and
b) obtaining 5-chloro-4-hydroxy-1-methyl-2-oxo-N-phenyl-1,2-dihydroquinoline-3-carboxamide from the reaction.

In an embodiment of the process, the reacting step is performed in a mixture of heptane and octane.

By any range disclosed herein, it is meant that all hundredth, tenth and integer unit amounts within the range are specifically disclosed as part of the invention. Thus, for example, 0.01 mg to 50 mg means that 0.02, 0.03 ... 0.09; 0.1,0.2... 0.9; and 1, 2... 49 mg unit amounts are included as embodiments of this invention.

A characteristic of a compound refers to any quality that a compound exhibits, e.g., peaks or retention times, as determined by 1H nuclear magnetic spectroscopy, mass spectroscopy, infrared, ultraviolet or fluorescence spectrophotometry, gas chromatography, thin layer chromatography, high performance liquid chromatography, elemental analysis, Ames test, dissolution, stability and any other quality that can be determined by an analytical method. Once the characteristics of a compound are known, the information can be used to, for example, screen or test for the presence of the compound in a sample.

As used herein, a "pharmaceutically acceptable" carrier or excipient is one that is suitable for use with humans and/or animals without undue adverse side effects (such as toxicity, irritation, and allergic response) commensurate with a reasonable benefit/risk ratio.

As used herein, "drug substance" refers to the active ingredient in a drug product, which provides pharmacological activity or other direct effect in the diagnosis, cure, mitigation, treatment, or prevention of disease, or to affect the structure or any function of the body of man or animals.

As used herein, "drug product" refers to the finished dosage form containing the drug substance as well as at least one pharmaceutically acceptable carrier.

As used herein, an "isolated" compound is a compound isolated from the crude reaction mixture following an affirmative act of isolation. The act of isolation necessarily involves separating the compound from the other known components of the crude reaction mixture, with some impurities, unknown side products and residual amounts of the other known components of the crude reaction mixture permitted to remain. Purification is an example of an affirmative act of isolation.

As used herein, a "composition" is distinct from a "pharmaceutical composition", and is substantially stable and unchanging over the course of a day. Thus, a composition as used herein is understood to be present in an inert environment. As used herein, a composition that is "free" of a chemical entity means that the composition contains, if at all, an amount of the chemical entity which cannot be avoided following an affirmative act intended to eliminate the presence of the chemical entity in the composition.

As used herein, "stability testing" refers to tests conducted at specific time intervals and various environmental conditions (e.g., temperature and humidity) to see if and to what extent a drug product degrades over its designated shelf life time. The specific conditions and time of the tests are such that they accelerate the conditions the drug product is expected to encounter over its shelf life. For example, detailed requirements of stability testing for finished pharmaceuticals are codified in 21 C.F.R §211.166. the entire content of which is hereby incorporated by reference.

As used herein, "about" in the context of a numerical value or range means ±10% of the numerical value or range recited or claimed.

Laquinimod is a small molecule having the following chemical structure:

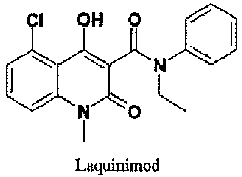

It is an oral immunomodulator which has demonstrated therapeutic effect in various experimental inflammatory/autoimmune animal models, such as Experimental Autoimmune Encephalomyelitis (EAE), an animal model for Multiple Sclerosis (MS), Dextran Sodium Solphate (DSS) induced colitis for Inflammatory Bowel Disease, Non-Obese Diabetic (NOD) mice for Type I Diabetes (IDDM), Experimental Autoimmune Neuritis (EAN) for Guillain-Barre Syndrome, Systemic Lupus Erythematosus (SLE), Multiple Sclerosis, lupus nephritis, lupus arthritis, Crohn's Disease and Rheumatoid arthritis. The therapeutic activity of Laquinimod in these models results from a variety of mechanistic effects, including reduction of leukocyte infiltration into target tissues by modulation of chemokine-mediated T-cell adhesion, modulation of cytokine balance, down regulation of MHC class II resulting in alteration of antigen presentation, and effects on dendritic cells subpopulations.

A pharmaceutically acceptable salt of laquinimod includes lithium, sodium, potassium, magnesium, calcium, manganese, copper, zinc, aluminum and iron. Salt formulations of laquinimod and the process for preparing the same are described, e.g., in U.S. Patent Application Publication No. 2005/0192315 and PCT International Application Publication No. WO 2005/074899, which are hereby incorporated by reference into this application.

A dosage unit may comprise a single compound or mixtures of compounds thereof. A dosage unit can be prepared for oral dosage forms, such as tablets, capsules, pills, powders, and granules.

Laquinimod can be administered in admixture with suitable pharmaceutical diluents, extenders, excipients, or carriers (collectively referred to herein as a pharmaceutically acceptable carrier) suitably selected with respect to the intended form of administration and as consistent with conventional pharmaceutical practices. The unit is preferably in a form suitable for oral administration. Laquinimod can be administered alone but is generally mixed with a pharmaceutically acceptable carrier, and co-administered in the form of a tablet or capsule, liposome, or as an agglomerated powder, Examples of suitable solid carriers include lactose, sucrose, gelatin and agar. Capsule or tablets can be easily formulated and can be made easy to swallow or chew; other solid forms include granules, and bulk powders. Tablets may contain suitable binders, lubricants, disintegrating agents, coloring agents, flavoring agents, flow-inducing agents, and melting agents. For instance, for oral administration in the dosage unit form of a tablet or capsule, the active drug component can be combined with an oral, non-toxic, pharmaceutically acceptable, inert carrier such as lactose, gelatin, agar, starch, sucrose, glucose, methyl cellulose, dicalcium phosphate, calcium sulfate, mannitol, sorbitol, microcrystalline cellulose and the like. Suitable binders include starch, gelatin, natural sugars such as glucose or beta-lactose, corn starch, natural and synthetic gums such as acacia, tragacanth, or sodium alginate, povidone, carboxymethylcellulose, polyethylene glycol, waxes, and the like. Lubricants used in these dosage forms include sodium oleate, sodium stearate, sodium benzoate, sodium acetate, sodium chloride, stearic acid, sodium stearyl fumarate, talc and the like. Disintegrators include, without limitation, starch, methyl cellulose, agar, bentonite, xanthan gum, croscarmellose sodium, sodium starch glycolate and the like.

Specific examples of the techniques, pharmaceutically acceptable carriers and excipients that may be used to formulate oral dosage forms of the present invention are described, e.g., in U.S. Patent Application Publication No. 2005/0192315, PCT International Application Publication Nos. WO 2005/074899, WO 2007/047863, and WO 2007/146248.

General techniques and compositions for making dosage forms useful in the present invention are described in the following references: 7 Modem Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James MeGinity. Eds., 1995); Aqueous Polymeric Coatings for Pharmaceutical Dosage Forms (Drugs and the Pharmaceutical Sciences, Series 36 (James MeGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modem Pharmaceutics Drugs and the Pharmaceutical Sciences, Vol. 40 (Gitbert S. Banker, Christopher T. Rhodes, Eds.). These references in their entireties are hereby incorporated by reference into this application.

### DELAQ as an Impurity

DELAQ(des-ethyl-laquinimod; 5-chloro-4-hydroxy-1-methyl-2-oxo-N-phenyl-1,2-dihydroquinoline-3-carboxamide), having the following chemical structure, is an undesirable synthetic by-product of laquinimod synthesis and a potential degradation by-product of laquinimod.

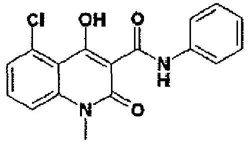

Any activity of DELAQ has not been fully characterized. Thus, it is generally desirable to minimize the amount of any impurity such as DELAQ in the laquinimod drug substance and the final drug product containing laquinimod.

DELAQ as an impurity in the laquinimod sodium drug substance is tested by a HPLC method and the specification for this impurity is provided as not more than 0.1%. The GMP drug substance batches of laquinimod sodium have been tested and the levels of DELAQ in these batches have been found to be less than 3 ppm.

Several analytical and bioanalytical methods were developed for determination of DELAQ concentrations. The current bioanalytical methods for DELAQ analysis in various matrices are based on LC-MS and have sensitivity at the low pg/mL plasma level.

This invention will be better understood by reference to the Experimental Details which follow, but those skilled in the art will readily appreciate that the specific experiments detailed are only illustrative of the invention as described more fully in the claims which follow thereafter.

### Experimental Details:

### Example 1: Preparation of DELAQ

### Synthesis of DELAQ from 5-chloro-1,2-dihydro-4-hydroxy-1-methyl-2-oxo-quinoline-3-carboxylic acid methyl ester

Preparation of 5-chloro-1,2-dihydro-4-hydrooxy-1-methyl-2-oxo-quinoline-3-carvboxylic acid methyl ester is described in **Example** 1 of U.S. Patent No. 7,560,557, entire content of which is hereby incorporated by reference.

5-chloro-1,2-dihydro-4-hydroxy-1-methyl-2-oxo-quinoline-3-carboxylic acid methyl ester (10.0 g), aniline (1.5 eq.), heptane (60 ml) and octane (60 ml) were mixed and heated. The volatiles, mainly heptane and formed methanol, were distilled off during 5 hours. After cooling to room temperature, the crystalline suspension was filtered and the crystals were washed with heptane and dried in vacuum to yield DELAQ (10.4g, 85% yield, >99% purity by HPLC).

### Example 2: Analysis of BELAQ as an Impuity in Laquinimod

DELAQ can be formed as an impurity in the manufacture of laquinimod, when starting material N-ethylaniline (NEA) contains aniline as an impurity. Therefore, the level of aniline in the starting material N-ethylaniline is monitored and N-ethylaniline is used for manufacture of laquinimod only if the aniline amount is less than 0.5%.

Doping starting material N-ethylaniline with aniline has resulted in a higher level of DELAQ in the Laquinimod sodium crude as shown in the table below.

| Doping Condition | Actual % by weight of aniline in NEA | % by weight of DELAQ in LAQ-Na crude | % by weight of DELAQ in LAQ-Na crystallized |
|---|---|---|---|
| Doping with 0.54% aniline and 1.08% diethyl aniline | 0.69 | 0.48 | ND |
| Doping with 0.54% aniline | 0.69 | 0.66 | ND |

The amount of aniline in the starting material N-ethylaniline is analyzed under the following HPLC conditions.
Column & Packing: Inertsil ODS-3V. Sµm, 4.6x250mm, GL Sciences
Guard column: Opti-Guard C 18, 1mm
Detection: UV at 240nm
Flow rate: 1.5mL/min
Injection volume: 50µL
Column temperature: 44°C
Autosampler temperature: 5°C
Mobile phase: 30% Solution A -70% Solution B (total concentration of ACN 55%)
Solution A: 800mL Ammonium acetate buffer - 200mL ACN
Solution B : 300mL Ammonium acetate buffer -700mL ACN
Buffer pH 7.0: Dissolve 7.7g of Ammonium acetate in 2440mL water and adjust with aqueous ammonia or glacial acetic acid to pH 7.0±0.05
Diluent A; Acetonitrile/Water 1:1
Diluent B: Mobile phase
Run time: At least 35 minutes

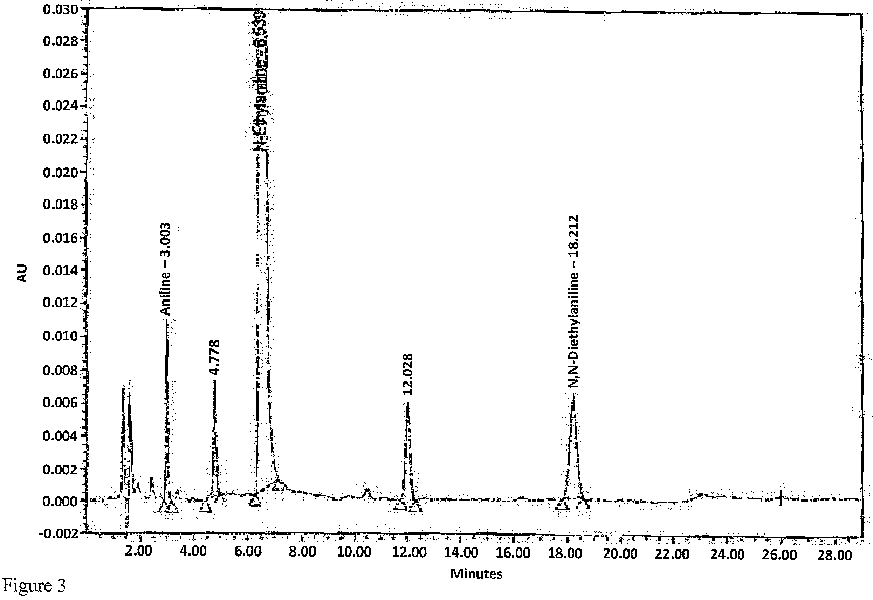

Samples of N-ethylaniline were analyzed for the presence of aniline using the HPLC method described above. Figure 3 is a HPLC chromatogram showing analytical results of a sample of N-ethylaniline under such HPLC conditions. As shown in Figure 3, aniline was present in the sample of N-ethylaniline at retention time of 3.003 minutes using the above HPLC method.

The DELAQ as an impurity in the laquinimod sodium drug substance has been monitored. A batch of the laquinimod sodium drug substance is approved for the preparation of final drug product only if the DELAQ impurity is not more than 0.1% using HPLC analysis.

The HPLC method used in analyzing the DELAQ impurity in the laquinimod sodium drug substance is based on a reversed phase HPLC, comprises a reverse phase column with high lipophilicity and very low silanol activity, mobile phase containing acetonitrile and aqueous ammonium acetate buffer, and a UV-vis detector, working at wavelength of 240 nm. The DELAQ impurity has been analyzed using HPLC under following conditions.

### Condition 1:

Column & Packing: Inertsil ODS-3V, 5µm, 4.6x250mm, GL Sciences
Guard column: Opti-Guard C 18, 1mm
UV detection: 240nm
Flow rate: 1.5mL/min
Injection volume: 5OµL
Column temperature: 40°C
Autosampler temperature: 5°C
Run time: 12 minutes
Mobile phase: 850mL ACN- 150mL Ammonium acetate buffer
Ammonium acetate buffer: Dissolve 7.7g of Ammonium acetate in 2000mL water and adjust to pH 7.0±0.05 with aqueous ammonia or glacial acetic acid.

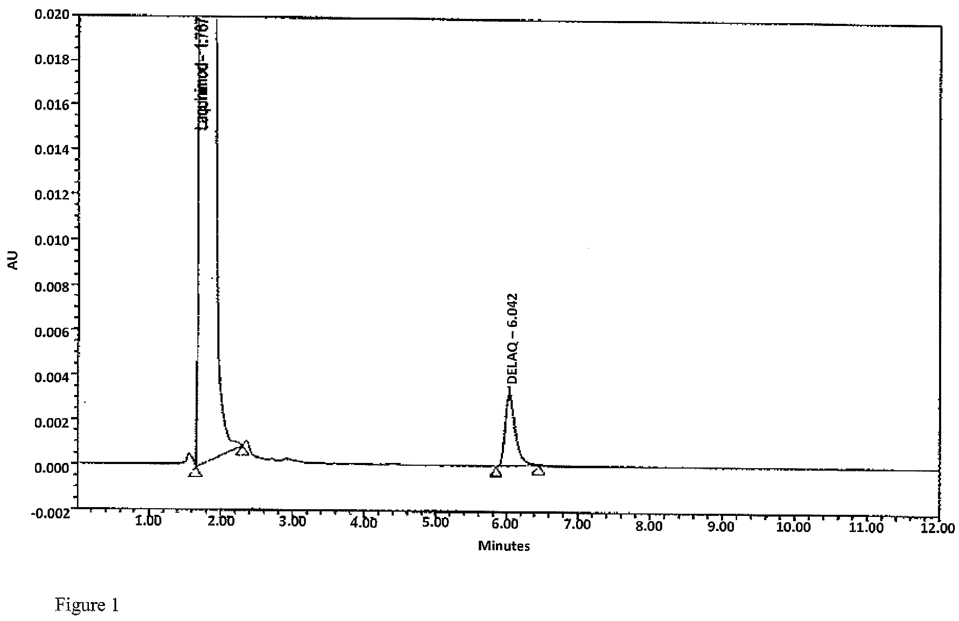

Samples of laquinimod drug substance were analyzed for the presence of DELAQ using the HPLC Condition 1 described above. Figure 1 is a HPLC chromatogram showing analytical results of a sample of laquinimod drug substance under such HPLC conditions. As shown in Figure 1, DELAQ was present in the sample of laquinimod drug substance at retention time of 6.042 minutes under HPLC Condition 1.

### Condition 2:

Column & Packing: Inertsil ODS-3V, 5µm, 4.6x250mm, GL Sciences
Guard column: Opti-Guard C18,1x10 mm
Detection: UV at 240 nm
Flow rate: 1.5 mL/min
Injection volume: 50µL
Column temperature: 40°C
Autosampler temperature: 5°C
Run time: 12 minutes
Mobile phase: Mix well 750 mL Acetonitrile and 250 mL Ammonium acetate buffer, degas before use
Ammonium acetate buffer: Dissolve 7.7g of Ammonium acetate in 2000 mL water and adjust to pH 7.0 ± 0.05 with aqueous ammonia or glacial acetic acid.

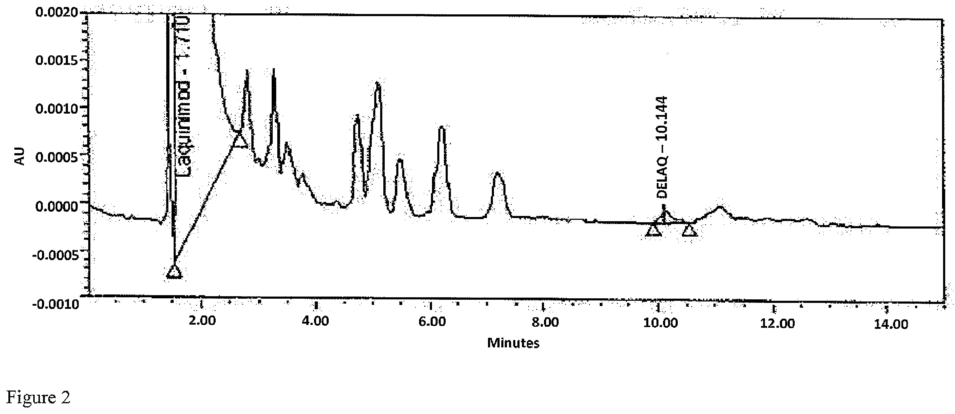

Samples of laquinimod drug substance were also analyzed for the presence of DELAQ using the HPLC Condition 2 described above. Figure 2 is a HPLC chromatogram showing analytical results of a sample of laquinimod drug substance under such HPLC conditions. As shown in Figure 2, DELAQ was present in the sample of laquinimod drug substance at retention time of 10.144 minutes under HPLC Condition 2.

The invention may be characterised by the following clauses;
1. A composition comprising a compound having the structure: 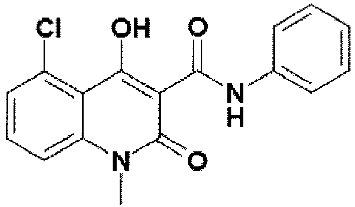 in an amount from more than 3ppm to less than 90 wt%, based on the total weight of the composition, and a carrier.
2. A pharmaceutical composition comprising a mixture of:
   a) laquinimod or a pharmaceutically acceptable salt thereof;
   b) at least one pharmaceutically acceptable carrier; and
   c) a compound having the structure: 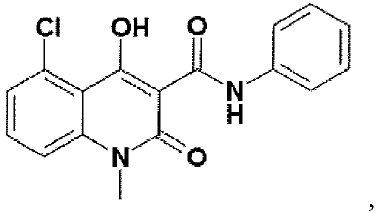 present in an amount less than 0.1% based on the combined weight of the compound and laquinimod,
3. The pharmaceutical composition of clause 2, wherein the compound is present in an amount less than 3 ppm based on the combined weight of the compound and laquinimod.
4. The pharmaceutical composition of clause 3, wherein the compound is present in an amount less than 2 ppm based on the combined weight of the compound and laquinimod.
5. The pharmaceutical composition of any of clauses 2-4 in the form of a tablet.
6. A process for preparing the pharmaceutical composition of any one of clauses 2-5, comprising:
   a) obtaining a batch of laquinimod or a pharmaceutically acceptable salt thereof;
   b) determining by apparatus the total amount of 5-chloro-4-hydroxy-1-methyl-2-oxo-N-phenyl-1,2-dihydroquinoline-3-carboxamide present in the batch of laquinimod or a pharmaceutically acceptable salt thereof; and
   c) preparing the pharmaceutical composition using the batch only if the batch is determined to have less than 0.10% by weight of 5-chloro-4-hydroxy-1-methyl-2-oxo-N-phenyl-1,2-dihydroquinoline-3-carboxamide.
7. A process for producing a validated batch of a pharmaceutical composition containing laquinimod or a pharmaceutically acceptable salt thereof and at least one pharmaceutically acceptable carrier for distribution comprising:
   a) obtaining a batch of the pharmaceutical composition;
   b) determining by apparatus the total amount of 5-chloro-4-hydroxy-1-methyl-2-oxo-N-phenyl-1,2-dihydroquinoline-3-carboxamide in a sample of the batch; and
   c) validating the batch for distribution only if the sample of the batch is determined to contain less than 0.1% by weight of 5-chloro-4-hydroxy-1-methyl-2-oxo-N-phenyl-1,2-dihydroquinoline-3-carboxamide relative to the combined weight of laquinimod and 5-chloro-4-hydroxy-1-methyl-2-oxo-N-phenyl-1,2-dihydroquinoline-3-carboxamide.
8. A process for producing laquinimod or a pharmaceutically acceptable salt thereof, comprising:
   a) obtaining a batch of N-ethylaniline;
   b) determining by apparatus the total amount of aniline in the batch of N-ethylaniline; and
   c) preparing laquinimod or a pharmaceutically acceptable salt thereof using the batch of N-ethylaniline only if the batch of N-ethylaniline is determined to have less than 0.5% aniline by weight.
9. A process for producing laquinimod or a pharmaceutically acceptable salt thereof, comprising:
   a) obtaining a batch of N-ethylaniline;
   b) purifying the batch of N-ethylaniline by separating aniline from the batch of N-ethylaniline; and
   c) preparing laquinimod or a pharmaceutically acceptable salt thereof using the purified batch of N-ethylaniline from step b).
10. A process for producing laquinimod or a pharmaceutically acceptable salt thereof, comprising:
   a) obtaining a batch of N-ethylaniline containing less than 0.5% aniline by weight; and
   b) preparing laquinimod or a pharmaceutically acceptable salt thereof using the batch of N-ethylaniline.
11. A process for preparing 5-chloro-4-hydroxy-1-methyl-2-oxo-N-phenyl-1,2-dihydroquinoline-3-carboxamide, comprising:
   a) reacting 5-chloro-1,2-dihydro-4-hydroxy-1-methyl-2-oxo-quinoline-3-carboxylic acid methyl ester and aniline under suitable conditions; and
   b) obtaining 5-chloro-4-hydroxy-1-methyl-2-oxo-N-phenyl-1,2-dihydroquinoline-3-carboxamide from the reaction.
12. The process of clause 11, wherein the reacting step is performed in a mixture of heptane and octane.

## Claims

1. A pharmaceutical composition comprising a mixture of:
a) laquinimod or a pharmaceutically acceptable salt thereof;
b) at least one pharmaceutically acceptable carrier; and
c) a compound having the structure: 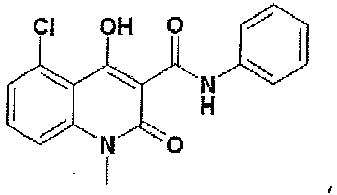 present in an amount less than 0.1% based on the combined weight of the compound and laquinimod.

2. The pharmaceutical composition of claim 1, wherein the compound is present in an amount less than 3 ppm based on the combined weight of the compound and laquinimod.

3. The pharmaceutical composition of claim 1, wherein the compound is present in an amount less than 2 ppm based on the combined weight of the compound and laquinimod.

4. The pharmaceutical composition of any of claims 1-3 in the form of a tablet.

5. A process for preparing the pharmaceutical composition of any one of claims 1 or 4, comprising:
a) obtaining a batch of laquinimod or a pharmaceutically acceptable salt thereof;
b) determining by apparatus the total amount of 5-chloro-4-hydroxy-1-methyl-2-oxo-N-phenyl-1,2-dihydroquinoline-3-carboxamide present in the batch of laquinimod or a pharmaceutically acceptable salt thereof; and
c) preparing the pharmaceutical composition using the batch only if the batch is determined to have less than 0.10% by weight of 5-chloro-4-hydroxy-1-methyl-2-oxo-N-phenyl-1,2-dihydroquinoline-3-carboxamide.

6. A process for producing a validated batch of a pharmaceutical composition containing laquinimod or a pharmaceutically acceptable salt thereof and at least one pharmaceutically acceptable carrier for distribution comprising:
a) obtaining a batch of the pharmaceutical composition;
b) determining by apparatus the total amount of 5-chloro-4-hydroxy-1-methyl-2-oxo-N-phenyl-1,2-dihydroquinoline-3-carboxamide in a sample of the batch; and
validating the batch for distribution only if the sample of the batch is determined to contain 5-chloro-4-hydroxy-1-methyl-2-oxo-N-phenyl-1,2-dihydroquinoline-3-carboxamide in an amount of less than 0.1% by weight relative to the combined weight of laquinimod and 5-chloro-4-hydroxy-1-methyl-2-oxo-N-phenyl-1,2-dihydroquinoline-3-carboxamide.

7. A process for producing laquinimod or a pharmaceutically acceptable salt thereof, comprising:
a) obtaining a batch of N-ethylaniline;
b) determining by apparatus the total amount of aniline in the batch of N-ethylaniline; and
c) preparing laquinimod or a pharmaceutically acceptable salt thereof using the batch of N-ethylaniline only if the batch of N-ethylaniline is determined to have less than 0.5% aniline by weight.

8. A process for producing laquinimod or a pharmaceutically acceptable salt thereof, comprising:
a) obtaining a batch of N-ethylaniline;
b) purifying the batch of N-ethylaniline by separating aniline from the batch of N-ethylaniline so that the batch of N-ethylaniline comprises less than 0.5% aniline by weight; and
c) preparing laquinimod or a pharmaceutically acceptable salt thereof using the purified batch of N-ethylaniline from step b).

9. A process for producing laquinimod or a pharmaceutically acceptable salt thereof, comprising:
a) obtaining a batch of N-ethylaniline containing less than 0.5% aniline by weight; and
b) preparing laquinimod or a pharmaceutically acceptable salt thereof; using the batch of N-ethylaniline.

10. The composition of claim 1 or the pharmaceutical composition of any one of claims 7.-4 in solid form.
